(19) Europäisches Patentamt
European Patent Office
Office européen des brevets

(11) Publication number: **0 353 755 A1**

(12) # EUROPEAN PATENT APPLICATION

(21) Application number: 89114364.6

(51) Int. Cl.$^4$: C07C 47/575

(22) Date of filing: 03.08.89

(30) Priority: 04.08.88 IT 2163988

(43) Date of publication of application: 07.02.90 Bulletin 90/06

(84) Designated Contracting States: BE CH DE ES FR GB IT LI NL

(71) Applicant: ISTITUTO GUIDO DONEGANI S.p.A.
Via Caduti del Lavoro
I-28100 Novara(IT)

(72) Inventor: Campolmi, Stefano
19, corso F.Cavallotti
F-28100 Novara(IT)
Inventor: Sogli, Loris
2, via Ragazzi del 99
F-28100 Novara(IT)
Inventor: Ungarelli, Raffaele
1, via Tanaro
F-28069 Trecate Novara(IT)

(74) Representative: Weinhold, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

(54) **Process for the production of veratral and related alkyl-aryl-ethers.**

(57) A process for the production of veratral and related alkyl-aryl-ethers, starting from vanillin or similar phenolic compounds, corresponding to the desired ethers, is described, wherein the starting compound is reacted with a base and an alkyl halide corresponding to the desired ether, in an initially aqueous or alcoholic reaction medium, the pH of this reaction medium being maintained constant at about 9.5 ± 0.5 (aqueous) or about 11 ± 0.5 (alcoholic), respectively.

EP 0 353 755 A1

## PROCESS FOR THE PRODUCTION OF VERATRAL AND RELATED ALKYL-ARYL-ETHERS

The present invention relates to a process for the preparation of alkyl-aryl-ethers, for instance veratral of formula:

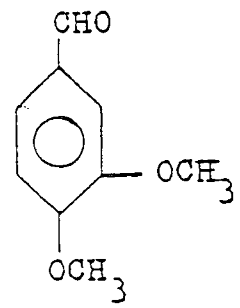

by alkylation with an alkyl halide, e.g. methyl halide, in the presence of a base, of the corresponding phenol (or naphthol), for instance vanillin of formula:

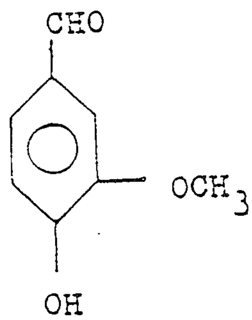

From Organic Synthesis, Collective Vol.II; 1943, page 619, it is known that the alkylation of vanillin may be achieved by forming the sodium salt of vanillin:

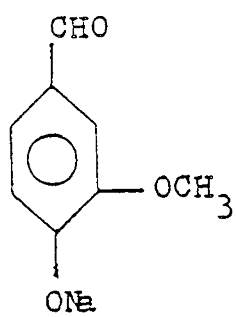

in an aqueous alkaline medium, followed by the alkylation of said salt with dimethylsulphate or a methyl halide. In said document it is stated, however, that a large excess of alkylating agent must be employed (the molar ratio alkylating agent/substrate being ≥2), since said agent simul taneously undergoes a hydrolysis reaction. Therefore, the consumption of alkylating agent and base are excessive, the final yields being equal to 80% at the most.

US-A-4,453,004 suggests to overcome said difficulties by completely eliminating any possible source of hydrolysis, that is, by carrying out the alkylation in the molten state, in the absence of water or any other kind of solvent. This procedure, however, results in a quite difficult separation of the desired product from the reaction mixture.

Furthermore, in said US-A some not very convenient and expensive compounds, such as, for instance, alkylphosphates and alkylsulphites, are recommended as alkylating agents.

There has now surprisingly been found a much simpler and more practical alkylation method which allows to work in an aqueous medium, with very high yields, while avoiding the hydrolysis of the alkylating agent, which alkylating agent is readily available and quite inexpensive.

In its broadest aspect, the present invention provides a process for the preparation of veratral and other similar alkyl-aryl-ethers by reacting vanillin or any other phenolic (naphtholic) compound corresponding to the desired ether with a base comprising an alkali metal or alkaline earth metal and with an alkyl halide corresponding to the desired ether, in an initially aqueous or alcoholic (or mixed aqueous-alcoholic) reaction medium.

Said process is characterized in that throughout the reaction the pH value of the reaction medium is kept constant at about 9.5 ± 0.5 (preferably at about 9.5 ± 0.25), when the reaction medium initially is aqueous, and at about 11 ± 0.5 when the reaction medium initially is alcoholic, depending on the initial amount of water and on the other reaction conditions.

An "initially" alcoholic reaction medium is referred to because during the reaction water is formed which water dilutes the alcohol; if an already diluted alcohol (with water) is used at the beginning, the pH has to be kept between the lowermost value (9.5 ± 0.5) and the uppermost value (11 ± 0.5).

It has also been found that the maximum yields are obtained when the pH is maintained constant around a value that results in the highest dissociation of the phenol (or naphthol) to be etherified.

Control of the pH may easily be achieved, for instance, by placing into the reactor the compound to be etherified (e.g. vanillin) together with a portion of the basic solution which is sufficient to reach the critical pH value, thereupon continuously introducing into the reactor the alkyl halide and at the same time replenishing the basic solution, thereby stabilizing the pH at the critical value.

In general it is advantageous to work with relatively small molar ratios of alkyl halide to substrate (e.g. vanillin), e.g. slightly higher than 1 (in general from about 1:1 to about 1.3:1). The end product (for instance veratral) is practically insoluble in the reaction medium and separates readily and continuously, thus favourably shifting the equilibrium of the reaction. This technique ensures considerable savings since for the recovery of the produced ether no organic solvents have to be employed, which solvents would require, downstream of the process, long and troublesome separation and recycling operations.

The critical choice of the pH value and the close control thereof render the process very selective and also lead to a satisfactory reaction rate. In fact, when working at pH values higher than the ones indicated above, a strong hydrolysis of the alkylating agent and further secondary reactions may be observed, while at lower pH values the extent of salification of the phenolic (or naphtholic) compound is reduced. Low concentrations of both the phenolate (naphtholate) and the alkylating agent in the solution drastically reduce the reaction rate, however.

The present invention also relates to the preparation of ethers different from veratral, by means of the the alkylation of a variety of phenolic and naphtholic compounds. Examples of suitable starting materials for the process of the present invention are:phenolic compounds of formula(I):

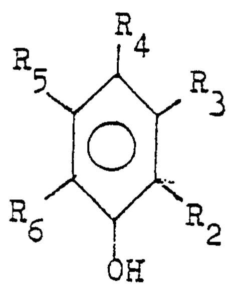

(I)

and naphtholic compounds of formulae (II) and (III):

(II) 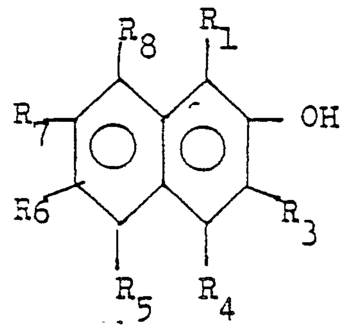 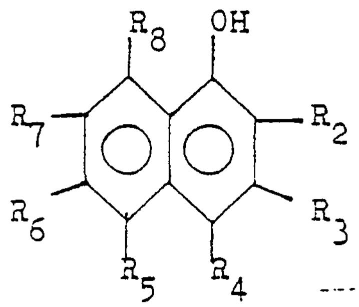 (III)

wherein $R_1$ to $R_8$, the same or different from each other, represent hydrogen, $NO_2$ or alkyl, cycloalkyl, aldehyde and alkoxy groups (linear or branched; saturated or unsaturated) having from 1 to 8 carbon atoms, and the like. Particularly preferred alkyl groups are those having 1 to 4 carbon atoms, i.e. methyl, ethyl, n- and i-propyl, n-butyl, i-butyl, sek-butyl and tert.-butyl; preferred cycloalkyl groups are cyclohexyl, cyclooctyl and cyclopentyl; alkoxy groups having 1 to 4 carbon atoms, e.g. methoxy and ethoxy, are also preferred.

In general not more than 3, particularly not more than 2 of $R_1$ to $R_8$ are different from hydrogen.

Particularly satisfactory results may be obtained by selecting the starting compound from vanillin, beta-naphthol, alpha-naphthol, para-nitrophenol, para-ethylphenol, isoeugenol and eugenol and/or by using alkyl bromides as the alkylating agents.

Besides in water, the alkylation of the phenolic or naphtholic OH-group may also be achieved in an alcoholic or hydroalcoholic solution (for instance: in $H_2O/C_2H_5OH$, $H_2O/C_3H_7OH$ etc.). In such a case, however, the pH value that ensures a high selectivity, i.e. close to 100%, will be higher.

The use of alcoholic solutions is, however, less preferred since the solvent is more expensive than water and the recovery of the ether produced is more complex in this case. The advantages of the present method, e.g. when the reaction medium is a purely aqueous medium, are particularly the following:

- the yield can be very high, even up to 100%, because of the absence of disturbing parallel reactions, and the consumption of alkylating agent is reduced to a level which is only slightly higher than the stoichiometric one;
- the obtained product, for instance, veratral, is practically insoluble in water and, therefore, can be removed by a simple solid/liquid separation operation (settling, filtration, centrifugation, etc.);
- no organic solvents are used which usually make the method more complicated and less competitive.

The detailed conditions for carrying out the process according to the present invention may be various. For the sake of brevity, only a few of them will be set forth in the following. The alkylation temperature may range from about 40 to about 200 °C, but preferably will be from about 60 to about 100 °C.

The alkylating agents preferably employed in the process of the present invention are of the formula $R_9Y$, wherein $R_9$ is a linear or branched alkyl group having 1 to 6, particularly 1 to 3, carbon atoms (e.g. methyl, ethyl, propyl) and Y is a halogen atom, preferably Cl, Br and I, particularly Br.

The alkyl halide (in particular methyl bromide) may be added semi-continuously to an aqueous solution containing the vanillin (or any other compound to be alkylated) and an initial amount of base (for instance: oxide, hydroxide, carbonate or bicarbonate of an alkali or alkaline earth metal, preferably of lithium, sodium, potassium, calcium or magnesium), in which solution the 'in situ' formation of the corresponding salt of the phenolic (or naphtholic) compound takes place.

As the reaction gradually proceeds, said (soluble) salt is transformed into an insoluble ether (for instance veratral) which separates from the aqueous medium. The initial amount of base employed is such as to bring the pH up to the desired value (9.5 ± 0.5) and the amount of base which is then introduced continuously, until the conversion is complete, obviously is the amount necessary for keeping the pH in the desired range. Said base may suitably be fed in the form of an aqueous solution (at a concentration of about 5 to about 30%, preferably of about 20% to about 25% by weight).

The process of the invention is illustrated by Figure 1, which in no way shall be construed as being limitative of the scope of the invention.

According to said Figure 1, vanillin (1) and an aqueous solution of potassium hydroxide (or carbonate) (2) are fed into a reaction zone (3) together with a methyl bromide re-cycle (8) coming from down-stream operations.

The veratral thus obtained is then converted, through a series of intermediate steps (4), not shown in the figure, into the hydrochloride of an L-amino acid [L-3-(3,4-dimethoxyphenyl)-2-methyl-alanine] and the latter, by reaction with hydrobromic acid (hydrogen bromide) (5), is then demethylated to L-methyl-dopa(3-hydroxy-alpha-methyl-L-tyrosine) (7).

The methyl bromide, a by-product of the demethylation, so far constituted an ecological problem, while according to the instant invention it is transformed, by means of recycle (8), into a very useful raw material for the synthesis of L-methyl-dopa which is an effective drug against hypertension.

The following examples are given in order to illustrate the present invention without, however, being a limitation thereof.

EXAMPLE 1:

45.6 of vanillin (0.3 mol) were placed into a 250 ml glass reactor and an aqueous solution of KOH (21% by weight) was added thereto in order to bring the pH up to a value of 9.5. Thereupon the reaction mixture was heated, under stirring, at 90 °C and then methyl bromide ($CH_3Br$) was bubbled through it, still at 90 °C, until the vanillin was completely converted (conversion time: 5.5 hours).

During the reaction, the pH was accurately maintained constant (pH = 9.5) by continuously adding said KOH solution. A total amount of 0.378 mol of KOH and 0.378 mol of $CH_3Br$ was added. The reaction mixture was then cooled down to room temperature and the separated veratral was filtered. The obtained filter cake was washed with 20 ml of deionized $H_2O$ and, after drying, there were obtained 49.85g of a white solid which showed the following characteristics:

| | |
|---|---|
| - melting point | 41 °C - 42 °C |
| - titre in veratral (GC) | 99.5% (corresponding to a yield of 99.5%). |

EXAMPLE 2:

45.6 g of vanillin (0.3 mol) were placed into the reactor of example 1; thereupon there was added a solution of KOH in methanol (30% by weight) until a pH value of 10.5 was reached. The reaction mixture was then heated at 60 °C, under constant stirring, whereafter methyl bromide ($CH_3Br$) was bubbled through it, still at 60 °C, until vanillin was completely converted (conversion time = 4.5 hours).

During the reaction, the pH was thoroughly kept constant (pH = 10.5) by continuously adding said alcoholic KOH solution. There was added a total of 0.363 mol of $CH_3Br$. The methanol was then distilled off and thereafter 20 ml of $H_2O$ and 30 ml of methylene chloride were added. The aqueous phase was separated and, after evaporation of the organic solvent, there were obtained 49.20 g of a white solid which showed the following characteristics:

| | |
|---|---|
| - melting point | 41 °C - 42 °C |
| - titre in veratral (GC) | 99.6% (equal to a yield of 98.3%). |

EXAMPLE 3:

45.6g of vanillin (0.3 mol) were placed into the reactor of example 1 and a solution of KOH in isopropyl alcohol (20% by weight) was added thereto until a pH value of 10.5 was reached. This reaction mixture was then heated under stirring to 75 °C, whereafter methyl bromide ($CH_3Br$) was bubbled through it, still at 75 °C, until the vanillin was completely converted (conversion time: 3.5 hours).

During the reaction, the pH was thoroughly kept constant (pH = 10.5) by continuous addition of said KOH solution. On the whole there were added 0.351 mol of KOH and 0.351 of $CH_3Br$. Thereafter, the alcohol was distilled off and thereupon 30 ml of deionized $H_2O$ and 30 ml of methylene chloride were added. The aqueous phase was separated and, after evaporation of the organic solvent, there were obtained 49.80 g of a white solid showing the following characteristics:

| | |
|---|---|
| - melting point | 42°C - 44°C |
| - titre in veratral (by gas-chromatography) | 99.1% (corresponding to a 99% yield). |

EXAMPLE 4 (comparison):

15.2g of vanillin (0.1 mol) were placed into the reactor of example 1. Then there was added an aqueous KOH solution (20% by weight) until the pH had reached a very high value (pH = 12).
The resulting reaction mixture was heated under stirring at 90°C and then methyl bromide ($CH_3Br$) was bubbled through it for 5 hours, still at 90°C. During the reaction the pH was thoroughly kept constant (pH = 11.5) by means of continuous addition of said KOH solution. On the whole there were added 0.216 mol of KOH and 0.220 mol of $CH_3Br$.

The reaction mixture was then allowed to cool down to room temperature, whereafter veratral was separated from said reaction mixture by filtration. The resulting cake was washed with 20 ml of deionized $H_2O$ and, after drying, there were obtained only 6.2g of a white solid showing the following characteristics:

| | |
|---|---|
| - melting point | 43°C - 45°C |
| - titre in veratral (by gas-chromatography) | 93.2% (corresponding to a 34.8% yield). |

This yield, which in comparison to that of example 1 is extremely poor, illustrates the criticality of the choice of the pH value during the reaction.

EXAMPLE 5:

32.8 g of eugenol (0.2 mol) were placed into the reactor of example 1, whereafter a KOH solution in methanol (30% by weight) was added until a pH value of 11.5 was reached. The resulting reaction mixture was heated, under stirring, at 60°C and then methyl bromide ($CH_3Br$) was bubbled through it, still at 60°C, until the eugenol was completely converted (conversion time: 3.5 hours).

During the reaction the pH was thoroughly kept constant at 11.5 by continuous addition of said KOH solution. On the whole, there were employed 0.216 mol of KOH and 0.216 mol of $CH_3Br$. The alcohol was distilled off and then there were added 30 ml of deionized $H_2O$ and 30 ml of methylene chloride. The aqueous phase was separated and, after evaporation of the organic solvent, there were obtained 35.2 g of a viscous oil.

| | |
|---|---|
| - titre in methyl-eugenol: (by gas-chromatography) | 100% (corresponding to a 98.8% yield). |

EXAMPLE 6:

14.4 g of beta-naphthol (0.1 mol) were placed into the reactor of example 1. Thereupon there was added a KOH solution in methanol (30% by weight) until a pH of 11.5 was reached. The resulting reaction mixture was heated, under stirring, at 60°C and methyl bromide ($CH_3Br$) was bubbled through it, still at 60°C, until the conversion of the beta-naphthol was complete (conversion time: 5.5 hours).
During the reaction, the pH was thoroughly maintained constant (i.e. pH = 11.5) by continuous addition of said KOH solution. On the whole, there were added 0.120 mol of KOH and 0.120 mol of $CH_3Br$. Thereupon the alcohol was distilled off and 30 ml of deionized $H_2O$ and 30 ml of methylene chloride were added. The aqueous phase was separated and, after evaporation of the organic solvent, there were obtained 15.70 g of a white solid that showed the following characteristics:

| | |
|---|---|
| - melting point: | 71 - 73 °C |
| - titre in beta-methoxynaphtalene (by gas-chromatography) | 99% (corresponding to a yield of 98.3 %) |

EXAMPLE 7:

13.9 g of p-nitro-phenol (0.1 mol) were placed into the reactor of example 1, and a KOH solution in methanol (30% by weight) was added thereto until a pH of 11.5 was reached. This resulting reaction mixture was heated, under stirring, at 60 °C, and then methyl bromide ($CH_3Br$) was bubbled through it, still at 60 °C, until the conversion of the p-nitrophenol was complete (conversion time: 6 hours).
During the reaction the pH was thoroughly maintained constant (i.e. pH = 11.5) by continuous addition of said KOH solution. On the whole, there were added 0.120 mol of KOH and 0.120 mol of $CH_3Br$. Thereafter the alcohol was distilled off and 30 ml of deionized $H_2O$ and 30 ml of methylene chloride were added. The aqueous phase was separated and, after evaporation of the organic solvent, there were obtained 15.20 g of a white solid having the following characteristics:

| | |
|---|---|
| - melting point: | 53 - 54 °C |
| - titre in p-nitro-anisol: (by gas-chromatography) | 99% (corresponding to a yield of 98.3%) |

EXAMPLE 8:

12.2 g of p-ethyl-phenol (0.1 mol) were placed into the reactor of example 1, whereupon a KOH solution in methanol (20% by weight) was added until a pH of 11.5 was reached. The resulting reaction mixture was heated, under stirring, at 60 °C and methyl bromide ($CH_3Br$) was bubbled through it, still at 60 °C, until the conversion of the p-ethyl-phenol was complete (conversion time: 5 hours).
During the reaction, the pH was thoroughly kept constant (i.e. pH = 11.5) by continuous addition of said KOH solution. On the whole, there were added 0.120 mol of KOH and 0.120 mol of $CH_3Br$.
The alcohol was distilled off and then there were added 30 ml of deionized $H_2O$ and 30 ml of methylene chloride. The aqueous phase was separated and, after evaporation of the organic solvent, there were obtained 13.50 g of a viscous oil.

| | |
|---|---|
| Titre in p-ethyl-anisol: (by gas-chromatography) | 99.6% (corresponding to a yield of 98.9%). |

## Claims

1. Process for the preparation of veratral and related alkyl-aryl-ethers by reaction of vanillin or the phenolic or naphtholic compound corresponding to the desired ether with an alkali metal or alkaline earth metal containing base and an alkyl halide corresponding to the desired ether, in an initially aqueous, alcoholic or hydro-alcoholic reaction medium, characterized in that the pH of the reaction medium is maintained constant, throughout the reaction, at about

a) pH = 9.5 ± 0.5, particularly 9.5 ± 0.25, when the reaction medium is initially aqueous;

b) pH = 11±0.5, when the reaction medium is initially alcoholic; and

c) pH = X ± 0.5, when the reaction medium is initially a mixture of water and alcohol, X being an intermediate value between 9.5 and 11.

2. Process according to claim 1, characterized in that the value X corresponds to the maximum dissociation of the phenol (or naphthol) to be etherified.

3. Process according to claim 1, alternative (a), characterized in that said base is fed in the form of an

aqueous solution and that said halide is an alkyl bromide.

4. Process according to claim 1, alternative (a), characterized in that the alkylation is carried out semicontinuously by first combining the compound to be alkylated and a first portion of the aqueous base and then continuously adding the alkyl halide and the rest of said aqueous base.

5. Process according to any one of the preceding claims, characterized in that the reaction temperature is from about 40 °C to about 200 °C, preferably from about 60 °C to about 100 °C.

6. Process according to any one of the preceding claims, characterized in that the reaction pressure is from about 1 to about 20 bar, preferably from about 1 to about 3 bar, provided said pressure is sufficient to maintain the reaction mixture in the liquid phase.

7. Process according to any one of the preceding claims, characterized in that the phenolic or naphtholic compound to be etherified is selected from

- phenolic compounds of formula (I):

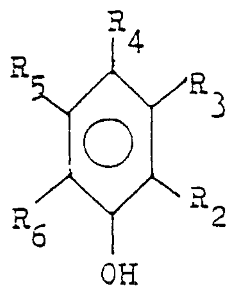

( I )

- naphtholic compounds of formula (II):

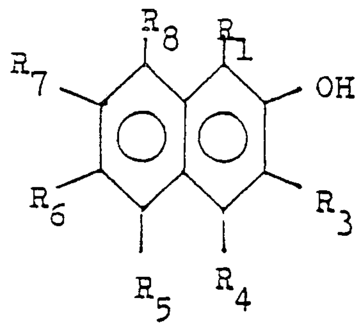

( II )

- naphtholic compounds of formula (III):

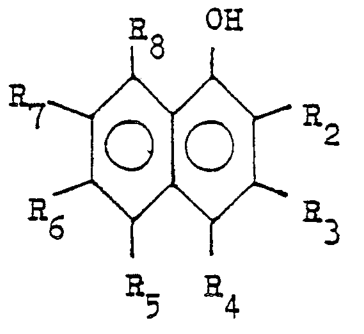

( III )

wherein $R_1$ to $R_8$, the same or different from each other, represent hydrogen, $NO_2$ or alkyl, cycloalkyl, aldehyde and alkoxy (linear or branched, saturated or unsaturated), having from 1 to 8 carbon atoms; particularly from vanillin, beta-naphthol, alpha-naphthol, para-nitrophenol, para-ethylphenol, iso-eugenol and eugenol.

8. Process according to any one of the preceding claims, wherein said base is selected from hydroxides, oxides, carbonates and bicarbonates of lithium, sodium, potassium, calcium and magnesium and mixtures of said compound, and particularly is KOH.

9. Process according to any one of the preceding claims, wherein said base is employed in the form of an aqueous solution, at a concentration of from 5 to about 30%, preferably of from about 20% to about 25% per weight.

10. Process according to any one of the preceding claims, wherein said alkyl halide is of the formula $R_9Y$ wherein $R_9$ is a linear or branched alkyl group having from 1 to 6 carbon atoms and Y is a halogen atom, preferably Cl, Br or I.

11. Process for the preparation of veratral, by reaction of vanillin with a alkali metal-based base and a methyl halide, characterized in that the reaction is conducted in the aqueous phase, maintaining the pH at a constant value of 9.5 ± 0.25 throughout the reaction and maintaining the temperature in the range of from about 60 to about 100 °C, and in that said methyl halide is methyl bromide ($CH_3Br$), coming as recycle from a known series of process steps by which veratral is converted into 3-hydroxy-alpha-methyl-L-tyrosine.

12. Process according to claim 11, wherein said alkali metal-based base is selected from hydroxides, oxides and carbonates of potassium and from mixtures of said bases and is employed in the form of an aqueous solution in a concentration of from about 20 to about 25% by weight, the molar ratio of methyl bromide to vanillin being from about 1:1 to about 1.3:1.

DO 3835

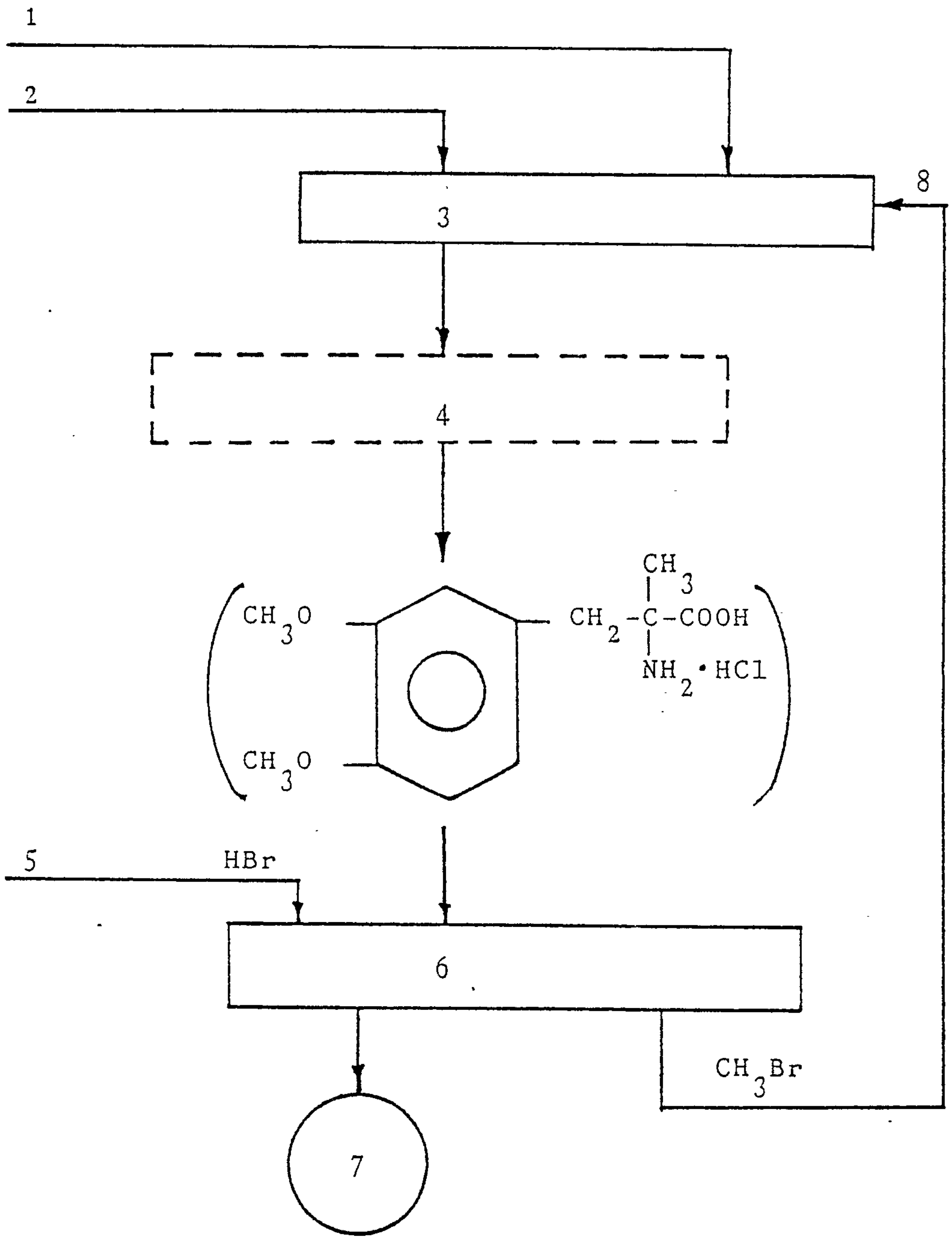


FIG. 1

European Patent Office

# EUROPEAN SEARCH REPORT

Application number

EP 89114364.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 2 496 803 (DONALD MC MILLAN) * Claim * | 1,8 | C 07 C 47/575 |
| D,A | US - A - 4 453 004 (RANDALL B. NELSON) * Claims 1,3-7 * | 1,8 | |
| A | DE - C - 214 153 (KALLE & CO.) * Claim; example * | 1,8,10 | |
| A | DE - B - 2 248 337 (F. HOFFMANN - LA ROCHE) * Claim 1 * | 1,8 | |
| A | GB - A - 1 337 370 (FUJI PHOTO FILM) * Claims 1,2,5; examples * | 1,5,8 | |
| A | US - A - 4 065 504 (DAVID MICHAEL FINDLAY) * Abstract * | 1,8 | TECHNICAL FIELDS SEARCHED (Int. Cl.4) C 07 C 47/00 C 07 C 43/00 C 07 C 41/00 |

The present search report has been drawn up for all claims

| Place of search | Date of completion of the search | Examiner |
|---|---|---|
| VIENNA | 03-11-1989 | REIF |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document